Europäisches Patentamt

European Patent Office

Office européen des brevets

(11) Veröffentlichungsnummer: **0 114 162**
**B1**

## (12) EUROPÄISCHE PATENTSCHRIFT

(45) Veröffentlichungstag der Patentschrift:
30.11.88

(51) Int. Cl.⁴ : **C 13 K 1/06, C 12 P 19/14**

(21) Anmeldenummer: **84890009.8**

(22) Anmeldetag: **10.01.84**

(54) Verfahren zum Aufbereiten von Stärke oder stärkehaltigen Rohstoffen.

(30) Priorität: 13.01.83 AT 109/83

(43) Veröffentlichungstag der Anmeldung:
25.07.84 Patentblatt 84/30

(45) Bekanntmachung des Hinweises auf die Patenterteilung: 30.11.88 Patentblatt 88/48

(84) Benannte Vertragsstaaten:
BE CH DE FR GB IT LI LU NL SE

(56) Entgegenhaltungen:
DE-A- 3 211 756
US-A- 4 243 750
US-A- 4 287 304
US-A- 4 361 651
CHEMICAL ABSTRACTS, Band 99, Nr. 13, September 1983, Seite 471, Nr. 103742s, Columbus, Ohio, US; & JP - A - 58 107 188 (NEW FUEL OIL DEVELOPMENT TECHNOLOGY RESEARCH ASSOC.) 18-12-1981

(73) Patentinhaber: **VOEST-ALPINE Aktiengesellschaft**
**Friedrichstrasse 4**
**A-1011 Wien (AT)**

(72) Erfinder: **Cvitas, Vilim, Dipl.-Ing.**
**Voltastrasse 29**
**A-4040 Lins (AT)**
Erfinder: **Faltejsek, Karl, Dipl.-Ing.**
**Lüfteneggerstrasse 6**
**A-4020 Linz (AT)**
Erfinder: **Hanke, Reinhart, Dipl.-Ing.**
**Annaberggasse 2**
**A-8700 Leoben (AT)**
Erfinder: **Treso, Bertalan**
**Kunigundenweg 11a**
**A-8707 Leoben (AT)**

(74) Vertreter: **Haffner, Thomas M., Dr. et al**
**Patentanwaltskanzlei Dipl.-Ing. Adolf Kretschmer Dr.**
**Thomas M. Haffner Schottengasse 3a**
**A-1014 Wien (AT)**

## Beschreibung

Die Erfindung bezieht sich auf ein Verfahren zum Aufbereiten von Stärke oder stärkehaltigen Rohstoffen für die Herstellung vergärbarer Zuckerlösungen, bei welchem die Rohstoffe mechanisch zerkleinert, mit Wasser aufgeschlämmt, thermisch bei Temperaturen von über 100 °C aufgeschlossen, verkleistert, und anschließend, nach einem Kühlen enzymatisch verzuckert werden.

Aus der US-A-4 361 651 ist bereits ein Verfahren bekanntgeworden, bei welchem Getreide, nach Abtrennung der Keime, zusammen mit Enzymen thermisch aufgeschlossen und anschließend auf die Verzuckerungstemperatur abgekühlt wird. Nach der Verzuckerung der Stärke werden nicht aufgeschlossene, in der Mischung vorhandene Faserbestandteile durch Sieben abgetrennt und in einem daran anschließenden Zentrifugier- bzw. Filterschritt die Proteinbestandteile aus der verzuckerten Mischung entfernt. Die Lösung wird in der Folge auf Gärtemperatur abgekühlt, fermentiert, die Hefe abgetrennt, und aus der Mischung verdünnter Alkohol abgezogen, worauf die verbleibende Lösung teilweise rückgeführt wird.

Aus der EP-A 0 044 428 ist ein derartiges Verfahren bereits bekannt geworden, bei welchem das stärkehaltige Material (z. B. Mais, Rüben, Rohr) durch mechanische Aufbereitung (Brechen, Sieben, Sichten) in eine stärkereiche (Speichergewebe) und stärkearme (Schalen) Fraktion getrennt wird. Die Trennung erfolgt unter Ausnutzung der unterschiedlichen Festigkeit und Dichte der beiden Gewebetypen. Die stärkearme Fraktion kann zu Futtermitteln weiterverarbeitet werden. Anschließend wird die stärkereiche Fraktion befeuchtet, wobei ein Wassergehalt von ca. 50 % eingestellt und thermisch aufgeschlossen wird. Der thermische Aufschluß des Zellgewebes und der Stärkekörner erfolgt unter Sattdampfbedingungen bei 110 bis 130 °C. Die aufgeschlossene Stärkefraktion wird mit Wasser weiter verdünnt und bei ca. 70 °C enzymatisch verzuckert. Die Verzuckerung erfolgt in einem Mischwerk, der Wassergehalt der Aufschlämmung beträgt hiebei 65 bis 80 %. Bei den bekannten Verfahren wurden nun bereits vor dem thermischen Aufschluß Enzyme für die Vergärung des Substrates zugesetzt und es wurde die erhaltene Aufschlämmung unmittelbar einer alkoholischen Gärung unterworfen. Nachteilig bei einer solchen Verfahrensweise ist die Tatsache, daß in einem derartigen Substrat während der Gärung die Gärungsgeschwindigkeit von Verdünnungs- und Diffusionsvorgängen in der Schlempe bestimmt wird, so daß sich eine hohe Gärzeit ergibt.

Die Erfindung zielt nun darauf ab, eine vergärbare Zuckerlösung herzustellen, welche in wesentlich kürzerer Zeit unmittelbar im Anschluß an die Aufbereitung zu Alkohol vergoren werden kann. Zur Lösung dieser Aufgabe besteht die Erfindung im wesentlichen darin, daß die verzuckerte Aufschlämmung auf Gärtemperatur oder eine geringfügig höhere Temperatur durch Verdünnen abgekühlt wird und daß die ungelösten Feststoffe anschließend vor dem Vergären abgetrennt werden. Durch die Abtrennung eines weiteren Teiles rückgewinnbarer Feststoffe nach der Verzuckerung läßt sich ein weiterer Rohstoff für die Futtermittelerzeugung gewinnen. Für die alkoholische Gärung kann durch die Verdünnung im Anschluß an die Verzuckerung unmittelbar die erforderliche Gärungstemperatur, beispielsweise ca. 35 °C, eingestellt werden. Die Abscheidung der Feststoffe nach dem Abkühlen kann beispielsweise durch Zentrifugieren oder Filtrieren erfolgen und gegebenenfalls durch eine Flockung der Feststoffe unterstützt werden. Für die nachfolgende Alkoholgärung wird somit eine praktisch feststofffreie Lösung erhalten, so daß der Gärprozeß durch inerte Feststoffe oder Adsorptionsvorgänge nicht beeinträchtigt wird.

In Verbindung mit einem nachgeschalteten Gärverfahren läßt sich bei der erfindungsgemäßen Verfahrensführung ein Großteil der erhaltenen Lösungen bzw. Hilfsstoffe im Kreislauf führen, so daß sich eine besonders hohe Wirtschaftlichkeit ergibt.

Insbesondere bei Herstellung eines Gärsubstrates durch Beladen von Hefe mit der auf erfindungsgemäße Weise hergestellten Zuckerlösung fällt vor dem Einbringen des Hefeschlammes in den Gärbehälter eine weitere Restzucker enthaltende Flüssigkeit an, welche erfindungsgemäß in vorteilhafter Weise für das Verdünnen der verzuckerten Aufschlämmung auf Gärtemperatur eingesetzt werden kann.

Die vom Hefeschlamm abgetrennte Lösung weist in der Regel 1 bis 8 Gew.% Zucker auf und läßt sich auch an anderen Stellen eines Verfahrens zur Herstellung von Äthanol aus stärkehaltigen Rohstoffen mit Vorteil wieder einsetzen. Hiebei kann so vorgegangen werden, daß die vom Hefeschlamm vor dem Einbringen desselben in den Reaktionsraum abgetrennte, Restzucker, Mengen von 1 bis 8 Gew.%, insbesondere 4 Gew.%, enthaltende Lösung, einer Aufbereitung der Zuckerlösung, zum Verdünnen und Kühlen einer verkleisterten Stärkelösung für das Verzuckern, zum Kühlen einer verzuckerten Lösung auf Gärtemperatur oder zum Herstellen stärkehaltiger Lösung, rückgeführt wird. Es kann somit die von der flotierten Hefe abgetrennte Zuckerlösung, welche bereits Gärtemperatur aufweisen kann, in der Stärkeaufbereitung und Stärkeverzuckerung zur Gewinnung der Zuckerlösung wieder eingesetzt werden. Im besonderen kann die genannte Lösung zur Befeuchtung des Stärkematerials vor der thermischen Konditionierung eingesetzt werden. Ebenso ist es möglich, die rückgeführte Lösung zur Verdünnung der thermisch aufgeschlossenen Stärke einzusetzen. Eine derartige Verdünnung ist für die enzymatische Verzuckerung von Stärkelösungen erforderlich, wobei gleichzeitig die Abkühlung des Materials von den Temperaturen der Verkleisterung bzw. thermischen Konditionierung, welche üblicherweise bei rund 120 °C liegen, auf rund 70 °C vorgenommen werden kann, wobei sich eine besonders günstige Temperatur für die enzymatische Verzuckerung ergibt. Ebenso kann die Restzucker enthaltende Lösung zur Abkühlung der verzuckerten Aufschlämmung auf Gärungstemperatur bzw. eine geringfügig über der Gärungstemperatur liegende Temperatur verwendet werden, so daß ein unmittelbar im Anschluß an ein derartiges Aufbereitungsverfahren von Stärke bzw. stärkehaltigen Rohstoffen angeschlossenes Gärverfahren mit hoher Wärmerückgewinnung bzw. Energieausbeute besonders wirtschaftlich durchgeführt werden kann. Bei Verwendung dieser Lösung für das Zudosieren des Polyelektrolyten zum Hefeschlamm kann die Polyelektrolytlösung annähernd auf Gärungstemperatur erwärmt werden, so daß auch hier besonders schonende Bedingungen und damit eine hohe Gärleistung sichergestellt werden kann.

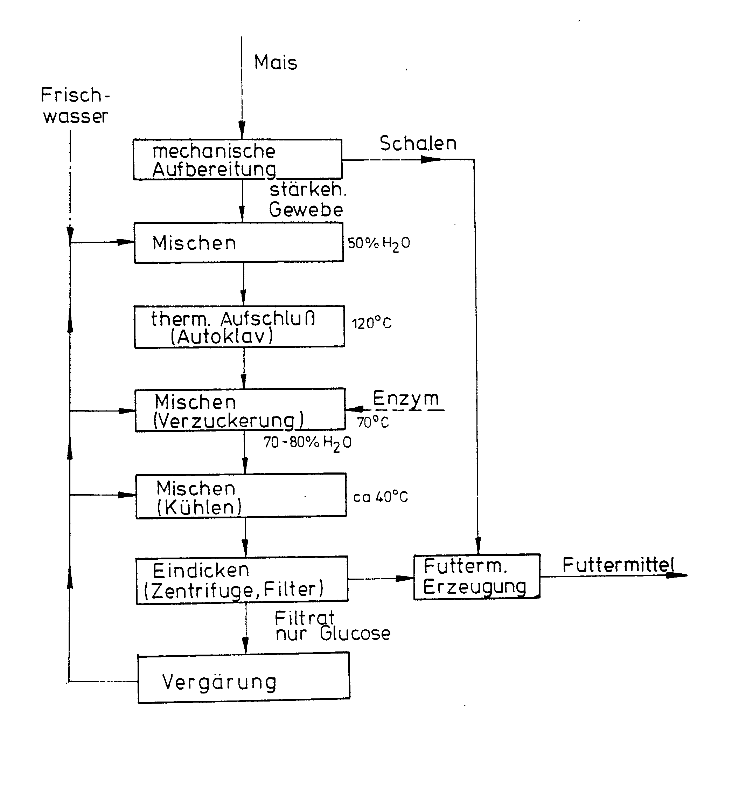

Die Erfindung wird nachfolgend an Hand eines in der Zeichnung dargestellten schematischen Flußdiagramms näher erläutert.

In dem Flußdiagramm, welches beispielsweise für Mais Gültigkeit hat, aber ohne nennenswerte Adaption auch für andere stärkehaltigen Rohstoffe Gültigkeit hat, folgt nach einer mechanischen Aufbereitung ein Mischen mit Wasser. Das für die Herstellung dieser Mischung erforderliche Wasser kann aus einem nachgeschalteten Gärverfahren rückgewonnen werden, wobei lediglich Austragverluste durch Frischwasser ersetzt werden müssen. Der thermische Aufschluß bzw. Verkleisterung erfolgt anschließend in einem Autoklaven bei etwa 120 °C. Im Anschluß an diesen thermischen Aufschluß ist ein Verdünnen und Abkühlen für die Verzuckerung erforderlich, wobei auch hier wiederum mit Vorteil eine Restzucker enthaltende Lösung aus nachfolgenden Verfahrensstufen, beispielsweise einer Gärung, eingesetzt werden kann. Anschließend an die Verzuckerung wird neuerlich mit Wasser gemischt und gekühlt, worauf ungelöste Feststoffe abgetrennt werden. Die ungelösten Feststoffe können zusammen mit den Schalen aus der mechanischen Aufbereitung einer Futtermittelerzeugung zugeführt werden. Das auf diese Weise erhaltene Filtrat stellt eine weitgehend reine Zuckerlösung dar, welche unmittelbar einer Vergärung unterworfen werden kann.

**Patentansprüche**

1. Verfahren zum Aufbereiten von Stärke oder stärkehaltigen Rohstoffen für die Herstellung vergärbarer Zuckerlösungen, bei welchem die Rohstoffe mechanisch zerkleinert, mit Wasser aufgeschlämmt, thermisch bei Temperaturen von über 100 °C aufgeschlossen, verkleistert und anschließend nach einem Kühlen enzymatisch verzuckert werden, dadurch gekennzeichnet, daß die verzuckerte Aufschlämmung auf Gärtemperatur oder eine geringfügig höhere Temperatur durch Verdünnen abgekühlt wird und daß die ungelösten Feststoffe anschließend vor dem Vergären abgetrennt werden.

2. Verfahren nach Anspruch 1, dadurch gekennzeichnet, daß bei Verwendung von mit Zucker beladener Hefe als Gärsubstrat die vom Hefeschlamm vor dem Einbringen desselben in den Reaktionsraum abgetrennte Restzucker, in Mengen von 1 bis 8 Gew.%, insbesondere 4 Gew.%, enthaltende Lösung einer Aufbereitung der Zuckerlösung, für das Verdünnen und Kühlen einer verkleisterten Stärkelösung für das Verzuckern, zum Kühlen einer verzuckerten Lösung auf Gärtemperatur oder zum Herstellen stärkehaltiger Lösungen rückgeführt wird.

**Claims**

1. Process for the treatment of starch or raw materials containing starch for the production of fermentable sugar solutions, in which the raw materials are crushed mechanically, are suspended with water, are hydrolyzed at a temperature of more than 100 °C, are gelatinized and than after cooling are enzymatically saccharified, characterized in that the saccharified suspension is cooled down by dilution to fermentation temperature or a slightly higher temperature and that the insoluble solid materials are separated thereafter before the fermentation.

2. Process according to claim 1, characterized in that by using yeast loaded with sugar as fermentation substrate the solution being separated from the yeast sludge before feeding the fermentation substrate into the reaction vessel and containing 1 to 8 % by weight, particularly 4 % by weight residual sugar, is recycled to a treatment of the sugar solution for either diluting and cooling of a gelatinized starch solution for saccharification or for cooling of a saccharified solution to fermentation temperature or for producing starch containing solutions.

**Revendications**

1. Procédé de traitement d'amidon ou de matières premières contenant de l'amidon pour la préparation de solutions sucrées fermentables, dans lequel les matières premières sont broyées mécaniquement, sont mis en suspension avec de l'eau, sont décomposées thermiquement à températures au-dessus de 100 °C, sont gélifiées et ensuite sont saccharifiées enzymatiquement après un refroidissement, caractérisé en ce que la suspension saccharifiée est refroidie par dilution à la température de fermentation ou une température légèrement plus haute et que les matières solides insolubles sont ensuite séparées avant la fermentation.

2. Procédé selon la revendication 1, caractérisé en ce que en usant levure chargée avec du sucre comme substrat de fermentation, la solution séparée du gâteau de levure avant de charger le substrat de fermentation dans le réacteur et contenant entre 1 et 8 % en poids, de préférence 4 % en poids, du sucre résiduel, est recyclée à un traitement de la solution sucrée soit pour la dilution et le refroidissement d'une solution d'amidon gélifiée pour la fermentation, soit pour le refroidissement d'une solution sucrée à la température de fermentation ou pour la préparation des solutions contenant de l'amidon.

Mais

Frischwasser

Schalen

mechanische
Aufbereitung

stärkeh.
Gewebe

Mischen — 50% $H_2O$

therm. Aufschluß
(Autoklav) — 120°C

Mischen
(Verzuckerung) — Enzym
70°C

70 - 80% $H_2O$

Mischen
(Kühlen) — ca 40°C

Eindicken
(Zentrifuge, Filter) — Futterm.
Erzeugung — Futtermittel

Filtrat
nur Glucose

Vergärung